(19) Europäisches Patentamt
European Patent Office
Office européen des brevets

(11) Veröffentlichungsnummer: **0 168 587**
**B1**

(12) # EUROPÄISCHE PATENTSCHRIFT

(45) Veröffentlichungstag der Patentschrift: **19.07.89**

(51) Int. Cl.$^4$: **C 07 C179/10**, C 07 C179/20, C 07 C179/22, C 07 C178/00

(21) Anmeldenummer: **85105978.2**

(22) Anmeldetag: **15.05.85**

(54) **Verfahren zur Herstellung von Monoperoxydicarbonsäuren und deren Salze.**

(30) Priorität: **20.07.84 DE 3418450**

(43) Veröffentlichungstag der Anmeldung: **22.01.86 Patentblatt 86/04**

(45) Bekanntmachung des Hinweises auf die Patenterteilung: **19.07.89 Patentblatt 89/29**

(84) Benannte Vertragsstaaten: **AT BE CH DE FR GB IT LI LU NL SE**

(56) Entgegenhaltungen:
**EP-A- 0 027 693**
**DE-B- 1 158 956**
**US-A- 2 377 038**
**US-A- 2 910 504**

(73) Patentinhaber: **Degussa Aktiengesellschaft**
**Weissfrauenstrasse 9**
**D-6000 Frankfurt am Main 1 (DE)**

(72) Erfinder: **Dankowski, Manfred, Dr.**
**Stifterstrasse 14**
**D-8757 Karlstein (DE)**

**EP 0 168 587 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Herstellung von Monoperoxydicarbonsäuren und deren Salze. Die Verwendung dieser Verbindungen als Bleichmittel in Waschmitteln ist seit langem bekannt.

In der AU-PS 417 480 wird die verbesserte Bleichwirkung von Monoperoxyphthalsäure in Gegenwart von $Mg^{2+}$-Ionen in der Bleichflotte beschrieben. Die FR-PS 2 129 034 betrifft die Herstellung von stabiler Monoperoxyphthalsäure aus Phthalsäureanhydrid und Wasserstoffperoxid in Gegenwart von 0,01 bis 1 Mol Magnesiumoxid, pro Mol Anhydrid. Aus dem dabei zumindest teilweise entstehenden Mg-Salz der Persäure wird diese durch Zusatz einer starken Mineralsäure ausgefällt. Eine alkalisch wirkende Magnesiumverbindung benötigt man ebenfalls gemäß der EP-B1-0 027 693, um die Oxydation von Phthalsäureanhydrid mit Wasserstoffperoxid durchzuführen und gleichzeitig das Magnesiumsalz der Monoperoxyphthalsäure zu gewinnen.

Die Herstellung von Monoperoxyphthalsäure wird in der Acta Chem. Scand. 12 (1958) 6, 1331 beschrieben. Um eine ausreichende Ausbeute zu erreichen, ist es notwendig, das Phthalsäureanhydrid bei einer Temperatur von unter — 5 °C mit der alkalischen Oxydationslösung umzusetzen. Die Persäure wird nach Versetzen der Lösung mit Schwefelsäure und Wasser durch Extraktion mit Äther abgetrennt.

Aufgabe der Erfindung ist ein Verfahren, um auf einfachem Wege Monoperoxydicarbonsäuren und deren Salze zu gewinnen.

Gegenstand der Erfindung ist ein Verfahren zur Herstellung von Monoperoxydicarbonsäuren (im folgenden Persäuren) und deren Alkali- bzw. Erdalkalisalze durch Oxidation der entsprechenden Anhydride mit Wasserstoffperoxid in einem die entstehenden Persäuren lösenden organischen Lösungsmittel, das dadurch gekennzeichnet ist, daß man die Oxidation in Gegenwart eines Anionenaustauschers vornimmt, nach erfolgter Umsetzung den Anionenaustauscher abtrennt und die Monoperoxydicarbonsäure aus der Lösung isoliert oder das entsprechende Monosalz durch Zusatz einer solchen Menge einer alkalisch wirkenden Alkali- oder Erdalkali-Verbindung ausfällt, die ausreicht, die resultierende nicht peroxidierte Carboxylgruppe zu neutralisieren.

Als Anhydride werden Verbindungen der folgenden Formeln eingesetzt:

a) Formel (I)

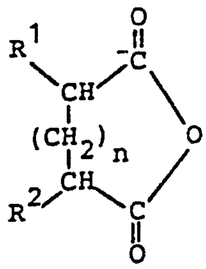

in der $R^1$ und $R^2$ gleich oder verschieden sind, Wasserstoff oder eine lineare oder verzweigte $C_1$-$C_{18}$ Alkylgruppe und n eine ganze Zahl von 0 bis 10, insbesondere 0 bis 2, bedeuten; bevorzugt sind die Anhydride der Glutarsäure, Bernsteinsäure und deren einfach alkylierte Derivate, deren Alkylgruppe 1 bis 18 Kohlenstoffatome aufweist.

b) Formel (II)

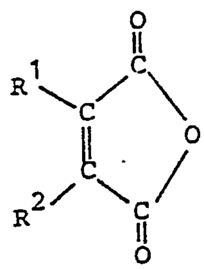

in der $R^1$ und $R^2$ dieselbe Bedeutung wie in Formel (I) haben; bevorzugt sind die Anhydride der Maleinsäure, Citraconsäure.

c) Formel III

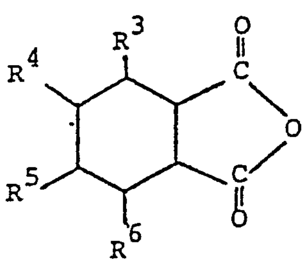

in der $R^3$, $R^4$, $R^5$, $R^6$ gleich oder verschieden sind und Wasserstoff eine lineare oder verzweigte $C_1$-$C_{10}$ Alkylgruppe, die Sulfonat oder Nitro-gruppe, Chlor oder Brom bedeuten oder $R^3$ und $R^4$, oder $R^4$ und $R^5$ oder $R^5$ und $R^6$ entfallen und zwischen den jeweiligen Ringkohlenstoffatomen eine Doppelbindung besteht; bevorzugt sind die Anhydride der Cyclohexan (1, 2) dicarbonsäure, 4-Methylcyclohexan-1,2-dicarbonsäure 4-Cyclohexen-1,2-dicarbonsäure.

d) Formel IV

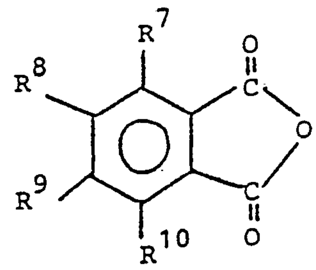

in der $R^7$, $R^8$, $R^9$ und $R^{10}$ gleich oder verschieden sind und Wasserstoff, eine lineare oder verzweigte $C_1$-$C_{10}$ Alkylgruppe, die Sulfonat- oder Nitrogruppe, Chlor oder Brom bedeuten; bevorzugt eingesetzt wird das Anhydrid der o-Phthalsäure.

Wasserstoffperoxid kommt in Form von wässrigen Lösungen zur Anwendung, deren Peroxidkonzentration von 20-99 %, bevorzugt 40 bis 60 % Gew.-% reicht.

Besonders geeignet ist die Ausführungsform, in der der Wassergehalt der Oxidationsmischung noch nicht zur Emulsionsbildung mit dem Lösungsmittel führt.

Wasserstoffperoxid wird in stöchiometrischem Verhältnis 1 : 1 zum Anhydrid eingesetzt. Ein leichter Überschuß von bis zu 0,2 Mol, bevorzugt 0,1 Mol Wasserstoffperoxid ist jedoch vorteilhaft.

Der Bereich für das Mengenverhältnis basischer Ionenaustauscher zu Anhydrid reicht von 0,1 g bis 50 g, bevorzugt 10 g bis 20 g, Ionenaustauscher je Mol Anhydrid. Verwendbar sind basische Ionenaustauscher, bevorzugt schwach basische, unter der Voraussetzung, daß sie oxidationsstabil sind. Diese Angabe kann man ohne Schwierigkeiten den Datenblättern der im Handel erhältlichen Ionenaustauscher entnehmen.

Die Verwendung der Ionenaustauscher ermöglicht im Gegensatz zu Verfahren nach dem Stand der Technik die kontinuerliche Herstellung der erfindungsgemäßen Persäuren, indem man z. B. die Reaktionsmischung auf einer zur Umsetzung ausreichenden Strecke mit dem Ionenaustauscher in Kontakt bringt.

Bei dem erfindungsgemäßen Verfahren entsteht ohne die Anwesenheit von nach dem Stand der Technik üblichen basischen Salzen wie z. B. Alkali- oder Erdalkalihydroxiden oder basischen Carbonaten und ohne Zusatz von Mineralsäure direkt die Monoperoxycarbonsäure.

Die Herstellung dieser Säuren wurde bisher dadurch erschwert, daß man den geeignetsten Zeitpunkt des Ansäuerns der Reaktionsmischung nicht genau vorausbestimmen konnte.

Wird ein Salz der Persäure gewünscht, fügt man nach dem Abtrennen des Ionenaustauschers der restlichen Reaktionslösung ein Alkali- oder Erdalkalioxid, -hydroxid oder -carbonat, bevorzugt Magnesiumverbindung, in einer Menge zu, die theoretisch ausreicht, um die verbleibende Carboxygruppe zu neutralisieren, bevorzugt in einem Überschuß von 1 %. Das besonders geeignete Magnesiumhydroxid wird in einem Molverhältnis von mindestens 1 : 2, bezogen auf die Persäure, eingesetzt. Besonders geeignet sind Magnesiumhydroxid/Persäure-Verhältnisse von 1 : 2 bis 1,1 : 2.

Im Fall der Monoperoxyphthalsäure entsteht das hydratisierte Salz

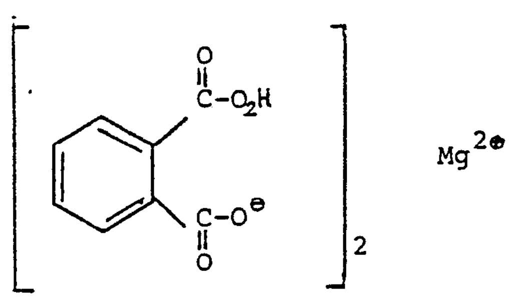

Die Reihenfolge der Zugabe von Anhydrid und Anionenaustauscher zum organischen Lösungsmittel ist nicht ausschlaggebend.

Es ist jedoch wichtig, daß die Temperatur bei der Umsetzung von Anhydrid und Wasserstoffperoxid kontrolliert wird. Die Reaktionstemperatur liegt in dem Bereich von — 10 °C bis 50 °C, bevorzugt 0 °C bis

20 °C, insbesondere 10 °C bis 20 °C. Als Reaktionszeit sind 5 min bis 5 h, bevorzugt 10 min bis 1 h, anzusetzen.

Die Reaktion zwischen der alkalischen Alkali- oder Erdalkaliverbindung und der gebildeten Monoperoxydicarbonsäure wird bevorzugt zwischen 15 °C und 20 °C mit einer Reaktionsdauer von 2 bis 20 min durchgeführt.

Das entstanden Salz der Persäure trennt man anschließend nach den Regeln der Technik z. B. über Filter oder Zentrifugen ab. Anschließend wird es bevorzugt mit nicht wässrigen Lösungsmitteln gewaschen, um die eventuell vorhandenen restlichen Anhydridmengen herauszulösen.

Als organisches Lösungsmittel setzt man aliphatische Ester mit geringem Molekulargewicht ein, bevorzugt Acetate und speziell Ethylacetat. Wichtig für die Auswahl ist ein gutes Lösevermögen des Lösungsmittels für die entstehende Persäure.

Das eingesetzte Anhydrid muß zumindest angelöst werden, die Alkali- oder Erdalkalisalze der Persäure lösen sich nicht in dem eingesetzten Lösungsmittel.

Bei dem in den nachfolgenden Beispielen eingesetzten Anionenaustauscher Lewatit® MP 62 (Bayer AG) handelt es sich um einen schwach basischen Ionenaustauscher.

Beispiel 1

25 g Phthalsäureanhydrid werden zu 150 ml Ethylacetat angelöst und auf 10 °C abgekühlt. Dann fügt man 3 g Lewatit® MP 62 hinzu. Anschließend werden innerhalb von 15 min 11,5 g 50 Gew.-% Wasserstoffperoxid bei gleicher Temperatur und unter Rühren eingetropft. Nun wird 1 h nachgerührt, wobei die Temperatur des Ansatzes auf ca. 20 °C steigt. Hiernach trennt man den Ionenaustauscher ab und gewinnt aus der Lösung 25,4 g Monoperoxyphthalsäure mit einem AO-Gehalt von 7,0 %.

Beispiel 2

25 g Phthalsäureanhydrid werden in 150 ml Ethylacetat angelöst und auf 10 °C abgekühlt. Dann fügt man 3 g Lewatit® MP 62 hinzu. Anschließend werden innerhalb von 15 min 11,5 g 50 Gew.-% Wasserstoffperoxid bei gleicher Temperatur und unter Rühren eingetropft. Nun wird 1 h nachgerührt, wobei die Temperatur des Ansatzes auf ca. 20 °C steigt. Hiernach trennt man den Ionentauscher ab und gewinnt nach Neutralisation mit 5 g Magnesiumhydroxid bei 20 °C in 15 min und Trocknen 31 g hydratisiertes Monoperoxyphthalsäure-mono-Mg-Salz mit einem AO-Gehalt von 5,1 %.

Beispiel 3

25 g Phthalsäureanhydrid werden in 150 ml Ethylacetat angelöst und auf 10 °C abgekühlt. Dann fügt man 3 g Lewatit® MP 62 hinzu. Anschließend werden innerhalb von 15 min 11,5 g 50 Gew.-% Wasserstoffperoxid bei gleicher Temperatur und unter Rühren eingetropft. Nun wird 3 h nachgerührt, wobei die Temperatur des Ansatzes auf ca. 20 °C steigt. Hiernach trennt man den Ionentauscher ab und gewinnt nach Neutralisation mit 5 g Magnesiumhydroxid bei 20 °C in 15 min und Trocknen 27 g hydratisiertes Monoperoxyphthalsäure-mono-Mg-Salz mit einem AO-Gehalt von 5,4 %.

Beispiel 4

25 g Phthalsäureanhydrid werden in 150 ml Ethylacetat angelöst und auf 20 °C temperiert. Dann fügt man 3 g Lewatit® MP 62 hinzu. Anschließend werden innerhalb von 15 min 11,5 g 50 Gew.-% Wasserstoffperoxid bei gleicher Temperatur und unter Rühren eingetropft. Nun wird 1 h bei dieser Temperatur nachgerührt. Hiernach trennt man den Ionentauscher ab und gewinnt nach Neutralisation mit 5 g Magnesiumhydroxid bei 20 °C in 15 min und Trocknen 23 g hydratisiertes Monoperoxyphthalsäure-mono-Mg-Salz mit einem AO-Gehalt von 5,2 %.

Beispiel 5

25 g Phthalsäureanhydrid werden in 100 ml Ethylacetat angelöst und auf 20 °C temperiert. Dann fügt man 3 g Lewatit® MP 62 hinzu. Anschließend werden innerhalb von 15 min 11,5 g 50 Gew.-% Wasserstoffperoxid bei gleicher Temperatur und unter Rühren eingetropft. Nun wird 1 h bei 30 °C nachgerührt. Hiernach trennt man den Ionentauscher ab und gewinnt nach Neutralisation mit 5 g Magnesiumhydroxid bei 20 °C in 15 min und Trocknen 25 g hydratisiertes Monoperoxyphthalsäure-mono-Mg-Salz mit einem AO-Gehalt von 5,2 %.

Beispiel 6

25 g Phthalsäureanhydrid werden in 150 ml Ethylacetat angelöst und auf 10 °C abgekühlt. Dann fügt man 1 g Lewatit® MP 62 hinzu. Anschließend werden innerhalb von 15 min 11,5 g 50 Gew.-% Wasserstoffperoxid bei gleicher Temperatur und unter Rühren eingetropft. Nun wird 1 h nachgerührt,

wobei die Temperatur des Ansatzes auf ca. 20 °C steigt. Hiernach trennt man der Ionentauscher ab und gewinnt nach Neutralisation mit 5 g Magnesiumhydroxid bei 20 °C in 15 min und Trocknen 25 g hydratisiertes Monoperoxyphthalsäure-mono-Mg-Salz mit einem AO-Gehalt von 5,4 %.

Beispiel 7

25 g Phthalsäureanhydrid werden in 150 ml Ethylacetat angelöst und auf 10 °C abgekühlt. Dan fügt man 3 g Lewatit® MP 62 hinzu. Anschließend werden innerhalb von 15 min 11,5 g 50 Gew.-% Wasserstoffperoxid bei gleicher Temperatur und unter Rühren eingetropft. Nun wird 1 h nachgerührt, wobei die Temperatur des Ansatzes auf ca. 20 °C steigt. Hiernach trennt man den Ionentauscher ab und gewinnt nach Neutralisation mit 3,4 g Magnesiumoxid bei 20 °C in 15 min und Trocknen 23 g hydratisiertes Monoperoxyphthalsäure-mono-Mg-Salz mit einem AO-Gehalt von 5,2 %.

Beispiel 8

25 g Phthalsäureanhydrid werden in 150 ml Ethylacetat angelöst und auf 10 °C abgekühlt. Dann fügt man 3 g Lewatit® MP 62 hinzu. Anschließend werden innerhalb von 15 min 11,5 g 50 Gew.-% Wasserstoffperoxid bei gleicher Temperatur und unter Rühren eingetropft. Nun wird 1 h nachgerührt, wobei die Temperatur des Ansatzes auf ca. 20 °C steigt. Hiernach trennt man den Ionentauscher ab und gewinnt nach Neutralisation mit 9,8 g basischem Magnesiumcarbonat bei 20 °C in 15 min und Trocknen 12 g hydratisiertes Monoperoxyphthalsäure-mono-Mg-Salz mit einem AO-Gehalt von 2,3 %.

Beispiel 9

25 g Phthalsäureanhydrid werden in 150 ml Ethylacetat angelöst und auf 10 °C abgekühlt. Dann fügt man 3 g Lewatit® MP 62 hinzu. Anschließend werden innerhalb von 15 min 20 g 30 Gew.-% Wasserstoffperoxid bei gleicher Temperatur und unter Rühren eingetropft. Nun wird 1 h nachgerührt, wobei die Temperatur des Ansatzes auf ca 20 °C steigt. Hiernach trennt man den Ionentauscher ab und gewinnt nach Neutralisation mit 5 g Magnesiumhydroxid bei 20 °C in 15 min und Trocknen 18 g hydratisiertes Monoperoxyphthalsäure-mono-Mg-Salz mit einem AO-Gehalt von 4,9 %.

Beispiel 10

25 g Phthalsäureanhydrid werden in 100 ml Ethylacetat angelöst und auf 10 °C abgekühlt. Dann fügt man 3 g Lewatit® MP 62 hinzu. Anschließend werden innerhalb von 15 min 15 g 40 Gew.-% Wasserstoffperoxid bei gleicher Temperatur und unter Rühren eingetropft. Nun wird 1 h nachgerührt, wobei die Temperatur des Ansatzes auf ca. 20 °C steigt. Hiernach trennt man den Ionentauscher ab und gewinnt nach Neutralisation mit 5 g Magnesiumhydroxid bei 20 °C in 15 min und Trocknen 25 g hydratisiertes Monoperoxyphthalsäure-mono-Mg-Salz mit einem AO-Gehalt von 5,3 %.

Beispiel 11

16,7 g Maleinsäureanhydrid werden in 150 ml Ethylacetat angelöst und auf 0 °C abgekühlt. Dann fügt man 3 g Lewatit® MP 62 hinzu. Anschließend werden innerhalb von 10 min 11,5 g 50 Gew.-% Wasserstoffperoxid bei gleicher Temperatur und unter Rühren zugetropft. Dann wird 1 h nachgerührt, wobei die Temperatur zwischen 0 und 5 °C gehalten wird. Hiernach trennt man den Ionentauscher ab und gewinnt aus der Lösung 16 g Monoperoxymaleinsäure mit einem AO-Gehalt von 10,8 %.

Beispiel 12

19,4 g Glutarsäureanhydrid werden in 150 ml Ethylacetat angelöst und auf 10 °C abgekühlt. Dann fügt man 3 g Lewatit® MP 62 hinzu. Anschließend werden innerhalb von 5 min 11,5 g 50 Gew.-% Wasserstoffperoxid bei gleicher Temperatur und unter Rühren zugetropft. Dann wird 1 h nachgerührt, wobei die Temperatur des Ansatzes auf ca. 20 °C steigt. Hiernach trennt man den Ionentauscher ab und gewinnt aus der Lösung 21 g Monoperoxyglutarsäure mit einem AO-Gehalt von 6,7 %.

Beispiel 13

26,2 g Cyclohexan-1,2-dicarbonsäureanhydrid werden in 150 ml Ethylacetat angelöst und auf 10 °C abgekühlt. Dann fügt man 3 g Lewatit® MP 62 hinzu. Anschließend werden innerhalb von 5 min 11,5 g 50 Gew.-% Wasserstoffperoxid bei gleicher Temperatur und unter Rühren eingetropft. Dann wird 1 h nachgerührt, wobei die Temperatur auf ca. 20 °C steigt. Hiernach trennt man den Ionentauscher ab und gewinnt aus der Lösung 25 g Monoperoxy-cyclohexan-1,2-dicarbonsäure mit einem AO-Gehalt von 4,0 %.

Beispiel 14

28,6 g 4-Methylcyclohexan-1,2-dicarbonsäureanhydrid werden in 150 ml Ethylacetat angelöst und auf 10 °C abgekühlt. Dann fügt man 3 g Lewatit® MP 62 hinzu. Anschließend werden innerhalb von 5 min 11,5 g 50 Gew.-% Wasserstoffperoxid bei gleicher Temperatur und unter Rühren eingetropft. Nun wird 1 h nachgerührt, wobei die Temperatur auf ca. 20 °C steigt. Hiernach trennt man den Ionentauscher ab und gewinnt aus der Lösung 28 g Monoperoxy-4-methylcyclohexan-1,2-dicarbonsäure mit einem AO-Gehalt von 4,0 %.

Beispiel 15

36,1 g Octylbernsteinsäureanhydrid werden in 150 ml Ethylacetat angelöst und auf 10 °C abgekühlt. Dann fügt man 3 g Lewatit® MP 62 hinzu. Anschließend werden innerhalb 5 min 11,5 g 50 Gew.-% Wasserstoffperoxid bei gleicher Temperatur und unter Rühren eingetropft. Nun wird 1 h nachgerührt, wobei die Temperatur auf ca. 20 °C steigt. Hiernach trennt man den Ionentauscher ab und gewinnt aus der Lösung 37 g Monoperoxy-2-octylbernsteinsäure mit einem AO-Gehalt von 3,5 %.

Beispiel 16

14,8 g Tetradecylbernsteinsäureanhydrid werden in 100 ml Ethylacetat angelöst und auf 10 °C abgekühlt. Dann fügt man 1 g Lewatit® MP 62 hinzu. Anschließend werden innerhalb von 10 min 3,4 g 50 Gew.-% Wasserstoffperoxid bei gleicher Temperatur und unter Rühren eingetropft. Nun wird 3 h nachgerührt, wobei die Temperatur auf ca. 20 °C steigt. Hiernach trennt man den Ionentauscher ab und gewinnt aus der Lösung 9 g Monoperoxy-2-tetradecylbernsteinsäure mit einem AO-Gehalt von 2,7 %.

**Patentansprüche**

1. Verfahren zur Herstellung von Monoperoxydicarbonsäuren und deren Alkali- bzw. Erdalkalisalze durch Oxidation der entsprechenden Anhydride mit Wasserstoffperoxid in einem die entstehenden Persäuren lösenden organischen Lösungsmittel, dadurch gekennzeichnet, daß man die Oxidation in Gegenwart eines Anionenaustauschers vornimmt, nach erfolgter Umsetzung den Anionenaustauscher abtrennt und die Monoperoxydicarbonsäure aus der Lösung isoliert oder das entsprechende Monosalz durch Zusatz einer Menge einer alkalisch wirkenden Alkali- oder Erdalkaliverbindung ausfällt, die ausreicht, die resultierende nichtperoxidierte Carboxylgruppe zu neutralisieren.

2. Verfahren gemäß Anspruch 1, dadurch gekennzeichnet, daß man Anhydride der Formeln (I) bis (IV) oxidiert : Formel (I)

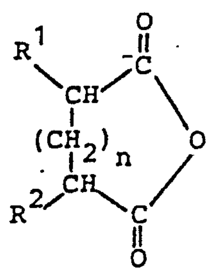

in der $R^1$ und $R^2$ gleich oder verschieden sind und Wasserstoff oder eine lineare oder verzweigte $C_1$-$C_{18}$ Alkylgruppe und n eine ganze Zahl von 0 bis 10 bedeuten ; Formel (II)

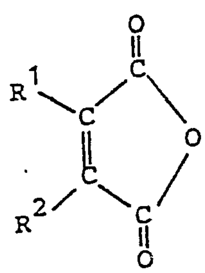

in der $R^1$ und $R^2$ gleich oder verschieden sind und Wasserstoff oder eine lineare oder verzweigte $C_1$-$C_{18}$ Alkylgruppe bedeuten ; Formel (III)

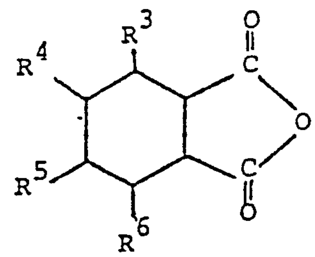

in der $R^3$, $R^4$, $R^5$, $R^6$ gleich oder verschieden sind und Wasserstoff eine lineare oder verzweigte $C_1$-$C_{10}$ Alkylgruppe, die Sulfonat oder Nitro-gruppe, Chlor oder Brom bedeuten oder $R^3$ und $R^4$, oder $R^4$ und $R^5$, oder $R^5$ und $R^6$ entfallen und zwischen den jeweiligen Ringkohlenstoffatomen eine Doppelbindung besteht ; Formel IV

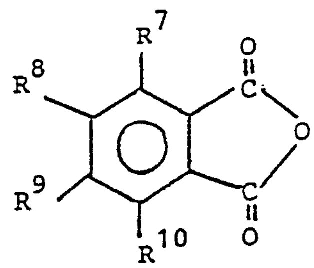

in der $R^7$, $R^8$, $R^9$ und $R^{10}$ gleich oder verschieden sind und Wasserstoff, eine lineare oder verzweigte $C_1$-$C_{10}$ Alkylgruppe, die Sulfonat- oder Nitrogruppe, Chlor oder Brom bedeuten.

3. Verfahren nach Anspruch 2, dadurch gekennzeichnet, daß man Phthalsäureanhydrid einsetzt.

4. Verfahren nach den Ansprüchen 1 bis 3, dadurch gekennzeichnet, daß man die Persäurebildung bei einer Temperatur von — 10 °C bis 50 °C, bevorzugt von 0 °C bis 20 °C, einer Reaktionszeit von 5 min bis 5 h, bevorzugt 10 min bis 1 h, einem Wasserstoffperoxid/Anhydrid-Verhältnis von 1 : 1 bis 1,2 : 1, bevorzugt 1,1 : 1, in Ethylacetat vornimmt.

5. Verfahren nach den Ansprüchen 1 bis 4, dadurch gekennzeichnet, daß man zur Bildung eines Salzes der Monoperoxycarbonsäure eine alkalisch wirkende anorganische Magnesiumverbindung verwendet.

6. Verfahren nach Anspruch 5, dadurch gekennzeichnet, daß man ein Magnesiumhydroxid/Persäure-Molverhältnis von 1 : 2 bis 1,1 : 2,0 wählt.

7. Verfahren nach den Ansprüchen 1 bis 5, dadurch gekennzeichnet, daß das Verhältnis Anionenaustauscher/Anhydrid von 0,1 g bis 50 g, bevorzugt 10 g bis 20 g, je Mol Anhydrid reicht.

**Claims**

1. Process for the preparation of monoperoxydicarboxylic acids and their alkali metal salts or alkaline earth metal salts by oxidizing the corresponding anhydrides with hydrogen peroxide in an organic solvent which dissolves the per-acids, formed, characterized in that the oxidation is carried out in the presence of an anion exchanger, the anion exchanger is removed after the completion of the reaction and the monoperoxydicarboxylic acid is isolated from the solution, or the corresponding mono-salt is precipitated by adding an amount of an alkali metal compound or alkaline earth metal compound having an alkaline reaction which is sufficient to neutralize the resulting, non-peroxidized carboxyl group.

2. Process according to Claim 1, characterized in that anhydrides of the formulae I to IV are oxidized : Formula (I)

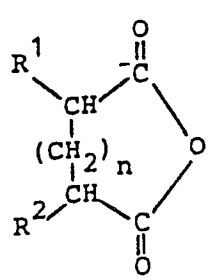

in which $R^1$ and $R^2$ are identical or different and denote hydrogen or a linear or branched $C_1$-$C_{18}$-alkyl group and n denotes an integer from 0 to 10 ; Formula (II)

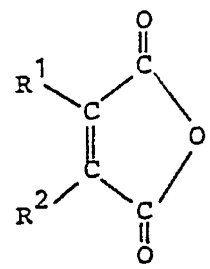

in which $R^1$ and $R^2$ are identical or different and denote hydrogen or a linear or branched $C_1$-$C_{18}$-alkyl group ; Formula (III)

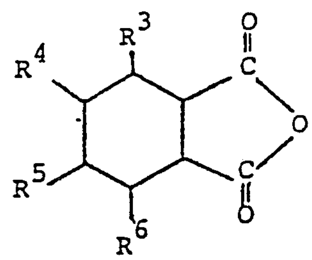

in which $R^3$, $R^4$, $R^5$ and $R^6$ are identical or different and denote hydrogen, a linear or branched $C_1$-$C_{10}$-alkyl group, the sulfonate or nitro group or chlorine or bromine, or $R^3$ and $R^4$, or $R^4$ and $R^5$, or $R^5$ and $R^6$ are not present and there is a double bond between the respective ring carbon atoms ; or Formula IV

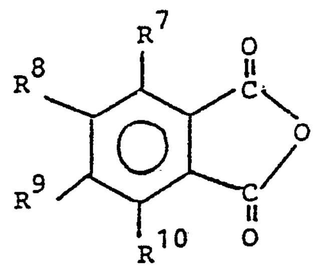

in which $R^7$, $R^8$, $R^9$ and $R^{10}$ are identical or different and denote hydrogen, a linear or branched $C_1$-$C_{10}$-alkyl group, the sulfonate or nitro group or chlorine or bromine.

3. Process according to Claim 2, characterized in that phthalic anhydride is employed.

4. Process according to Claims 1 to 3, characterized in that the formation of the per-acid is carried out at a temperature from —10 °C to 50 °C, preferably from 0 °C to 20 °C, in a reaction time from 5 minutes to 5 hours, preferably 10 minutes to 1 hour, at a hydrogen peroxide/anhydride ratio of 1 : 1 to 1.2 : 1, preferably 1.1 : 1, and in ethyl acetate.

5. Process according to Claims 1 to 4, characterized in that an inorganic magnesium compound having an alkaline reaction is used to form a salt of the monoperoxycarboxylic acid.

6. Process according to Claim 5, characterized in that the magnesium hydroxide/per-acid molar ratio selected is from 1 : 2 to 1.1 : 2.0.

7. Process according to Claims 1 to 5, characterized in that the anion exchanger/anhydride ratio extends from 0.1 g to 50 g, preferably 10 g to 20 g, per mole of anhydride.

## Revendications

1. Procédé pour la préparation d'acides monoperoxydicarboxyliques et de leurs sels alcalins ou alcalino-terreux, par oxydation des anhydrides correspondants avec le peroxyde d'hydrogène dans un solvant organique dissolvant les peracides formés, caractérisé en ce que l'on effectue l'oxydation en présence d'un échangeur d'anions, une fois la réaction terminée, on sépare l'échangeur d'anions et on isole l'acide monoperoxydicarboxylique à partir de la solution ou on fait précipiter l'hydrogénosel correspondant par addition d'une quantité d'un composé alcalinisant alcalin ou alcalino-terreux qui est suffisante pour neutraliser le groupe carboxyle non peroxydé résultant.

2. Procédé selon la revendication 1, caractérisé en ce que l'on oxyde des anhydrides de formules (I) à (IV) : Formule (I) :

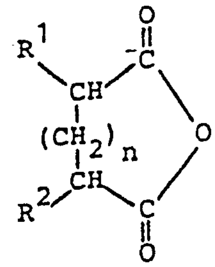

dans laquelle $R^1$ et $R^2$ sont identiques ou différents, et représentent un atome d'hydrogène ou un groupe alkyle en $C_1$-$C_{18}$ linéaire ou ramifié, et n représente un nombre entier valent de 0 à 10 ; Formule (II) :

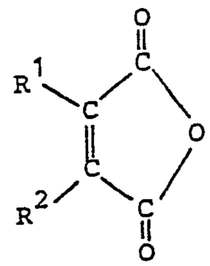

dans laquelle $R^1$ et $R^2$ sont identiques ou différents, et représentent un atome d'hydrogène ou un groupe alkyle en $C_1$-$C_{18}$ linéaire ou ramifié ; Formule (III)

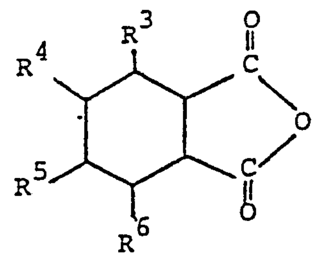

dans laquelle $R^3$, $R^4$, $R^5$, $R^6$ sont identiques ou différents, et représentent un atome d'hydrogène ou un groupe alkyle en $C_1$-$C_{10}$ linéaire ou ramifié, le groupe sulfonate ou nitro, un atome de chlore ou de brome, ou $R^3$ et $R^4$, ou $R^4$ et $R^5$, ou $R^5$ et $R^6$ sont absents et il y a une double liaison entre les atomes de carbone cycliques respectifs ; Formule (IV) :

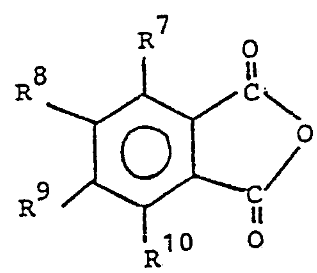

dans laquelle : $R^7$, $R^8$, $R^9$ et $R^{10}$ sont identiques ou différents et représentent un atome d'hydrogène, un groupe alkyle en $C_1$-$C_{10}$ linéaire ou ramifié, le groupe sulfonate ou nitro, un atome de chlore ou de brome.

3. Procédé selon la revendication 2, caractérisé en ce que l'on utilise l'anhydride phtalique.

4. Procédé selon l'une quelconque des revendications 1 à 3, caractérisé en ce que l'on effectue la formation de peracide à une température de —10 °C à 50 °C, de préférence de 0 °C à 20 °C, pendant une durée de réaction allant de 5 minutes à 5 heures, de préférence de 10 minutes à 1 heure, avec une proportion de peroxyde d'hydrogène/anhydride allant de 1 : 1 à 1,2 : 1, de préférence de 1,1 : 1, dans de l'acétate d'éthyle.

5. Procédé selon l'une quelconque des revendications 1 à 4, caractérisé en ce que, pour la formation d'un sel de l'acide monoperoxycarboxylique, on utilise un composé minéral de magnésium, alcalinisant.

6. Procédé selon la revendication 5, caractérisé en ce que l'on choisit un rapport molaire hydroxyde de magnésium/peracide allant de 1 : 2 à 1,1 : 2,0.

7. Procédé selon l'une quelconque des revendications 1 à 5, caractérisé en ce que la proportion échangeur ionique/anhydride va de 0,1 g à 50 g, de préférence de 10 g à 20 g, par mole d'anhydride.